(19) Europäisches Patentamt
European Patent Office
Office européen des brevets

(11) **EP 3 482 784 A1**

(12) **EUROPEAN PATENT APPLICATION**

(43) Date of publication:
**15.05.2019 Bulletin 2019/20**

(51) Int Cl.:
***A61M 1/16*** (2006.01)

(21) Application number: **18204839.7**

(22) Date of filing: **07.11.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.11.2017 US 201762583356 P**
**01.10.2018 US 201816148383**

(71) Applicant: **Medtronic, Inc.**
**Minneapolis, Minnesota 55432 (US)**

(72) Inventors:
• **MAZACK, Michael J.**
**Roseville, MN Minnesota 55113 (US)**
• **GERBER, Martin T.**
**Maple Grove, MN Minnesota 55369 (US)**
• **HOBOT, Christopher M.**
**Rogers, MN Minnesota 55374 (US)**

(74) Representative: **Maschio, Antonio**
**Maschio & Soames IP Limited**
**30 Carlton Crescent**
**Southampton SO15 2EW (GB)**

(54) **PATIENT BUN ESTIMATOR FOR SORBENT HEMODIALYSIS**

(57) The invention relates to systems and methods for estimating a patient urea level at any arbitrary time during dialysis treatment. The systems and methods use either one or more urea sensors or any two of a pH sensor, ammonia sensor, and ammonium sensor to determine an amount of urea removed by a dialysate regeneration system. The systems and methods use the amount of urea removed by the dialysate regeneration system to estimate the patient urea level.

EP 3 482 784 A1

**Description**

CROSS-REFERENCE TO RELATED APPLICATIONS

This application claims the benefit of and priority to U.S. Provisional Patent Application No. 62/583,356 filed November 8, 2017, the entire disclosure of which is incorporated by reference herein.

FIELD OF THE INVENTION

The invention relates to systems and methods for estimating a patient urea level at any arbitrary time during dialysis treatment. The systems and methods use either one or more urea sensors or any two of a pH sensor, ammonia sensor, and ammonium sensor to determine an amount of urea removed by a dialysate regeneration system. The systems and methods use the amount of urea removed by the dialysate regeneration system to estimate the patient urea level.

BACKGROUND

An important solute in hemodialysis treatment is urea which can be used as a marker for dialysis adequacy. Known systems and methods generally use blood samples from the patient prior to dialysis, which are then analyzed using a blood gas analyzer to measure BUN.
Hence, there is a need for systems and methods that can estimate patient urea levels without the need to draw and analyze blood from the patient prior to each dialysis system. There is a further need for systems and methods that can measure patient urea level without the need for a blood-gas analyzer or a plate-based assay. There is a need for systems and methods using sensors in a dialysate flow path to estimate the patient urea level, which can be used to calculate the adequacy of dialysis treatment.

SUMMARY OF THE INVENTION

The first aspect of the invention is drawn to a dialysis system. In any embodiment, the dialysis system can comprise a dialysate flow path fluidly connectable to a dialyzer; the dialysate flow path having a dialysate regeneration system comprising urease; either a urea sensor or at least two sensors selected from: ammonia sensors, ammonium sensors, and pH sensors; and a processor; the processor in communication with the urea sensor, ammonia sensor, ammonium sensor, and/or pH sensor; the processor programmed to determine an amount of urea removed by the dialysate regeneration system; the processor further programmed to estimate a patient urea level from either one or more lookup tables or a mathematical model based on the amount of urea removed by the dialysate regeneration system;

$$\frac{dM_{P,i}}{dt} = G_{P,i} - (Ind)J_i(V_P, M_{P,i}, C_{Di,i}) + R_{P,i}$$

the mathematical model using solutions to a formula: , wherein $M_{p,i}$ is a mass of a species "i" in the patient, $G_{p,i}$ is a generation rate of the of the species "i" in the patient, $J_i$ is a total mass transfer rate of species "i" from the patient into a dialysate, $C_{Di,i}$ is a concentration of species "i" in a regenerated dialysate when the regenerated dialysate enters the dialyzer, $R_{P,i}$ is a production rate of species "i" due to chemical reactions, and *Ind* is a binary indicator variable for dialysis therapy with *Ind* = 0 if dialysis is not occurring, and *Ind* = 1 if dialysis is occurring.

In any embodiment, the mathematical model can use a formula: $CBi_{Urea}^{tCrit} = \left(\frac{QDi}{D_{Urea}}\right)(CDo_{Urea} - CDi_{Urea}) + CDi_{Urea}$; wherein $CBi_{Urea}^{tCrit}$ is the patient urea level at time *tCrit*; *QDi* is a dialysate flow rate entering the dialyzer; $D_{Urea}$ is a dialysance of urea; $CDo_{Urea}$ is a urea concentration in the dialysate exiting the dialyzer; and $CDi_{Urea}$ is a urea concentration in the dialysate entering the dialyzer.
In any embodiment, the dialysate regeneration system can comprise one or more sorbent cartridges.
In any embodiment, the sensors can comprise a first urea sensor located upstream of the dialysate regeneration system and an optional second urea sensor located downstream of the dialysate regeneration system.
In any embodiment, the sensors can comprise at least two of an ammonia sensor, an ammonium sensor, and a pH sensor located downstream of the urease and upstream of an ammonium and/or ammonia exchange material.
In any embodiment, the sensors can be part of a combined pH, ammonium, and/or ammonia sensor.

In any embodiment, the processor can be further programmed to estimate a urea reduction ratio based on a urea level of the patient at a beginning of a dialysis session and a urea level of the patient at an end of the dialysis session.

In any embodiment, the processor can be further programmed to estimate a urea reduction ratio at an arbitrary time during treatment.

In any embodiment, the dialysis system can have a single urea sensor upstream of the dialysate regeneration system.

In any embodiment, the processor can be programmed to estimate the patient urea level at a beginning of a dialysis session.

The features disclosed as being part of the first aspect of the invention can be in the first aspect of the invention, either alone or in combination.

The second aspect of the invention relates to a method. In any embodiment, the method can comprise (a) initiating a dialysis session for a patient using a dialysis system having a dialysate regeneration system comprising urease in a dialysate flow path; (b) determining an amount of urea removed by the dialysate regeneration system based on data received from either one or more urea sensors or at least two sensors selected from: urea sensors, ammonia sensors, ammonium sensors, and pH sensors; and (c) estimating a patient urea level from a mathematical model or lookup table based on the amount of urea removed by the dialysate regeneration system; the mathematical model using solutions to a formula:

$$\frac{dM_{P,i}}{dt} = G_{P,i} - (Ind)J_i(V_P, M_{P,i}, C_{Di,i}) + R_{P,i}$$

, wherein $M_{p,i}$ is a mass of a species "$i$" in the patient, $G_{p,i}$ is a generation rate of the of the species "$i$" in the patient, $J_i$ is a total mass transfer rate of species "$i$" from the patient into a dialysate, $C_{Di,i}$ is a concentration of species "$i$" in a regenerated dialysate when the regenerated dialysate enters the dialyzer, $R_{P,i}$ is a production rate of species "$i$" due to chemical reactions, and $Ind$ is a binary indicator variable for dialysis therapy with $Ind = 0$ if dialysis is not occurring, and $Ind = 1$ if dialysis is occurring.

In any embodiment, the mathematical model can use a formula: $CBi_{Urea}^{tCrit} = \left(\frac{QDi}{D_{Urea}}\right)(CDo_{Urea} - CDi_{Urea}) + CDi_{Urea};$ wherein $CBi_{Urea}^{tCrit}$ is the patient urea level at time $tCrit$; $QDi$ is a dialysate flow rate entering the dialyzer; $D_{Urea}$ is a dialysance of urea; $CDo_{Urea}$ is a urea concentration in a dialysate exiting the dialyzer; and $CDi_{Urea}$ is a urea concentration in the dialysate entering the dialyzer.

In any embodiment, the sensors can comprise a first urea sensor located upstream of the dialysate regeneration system and an optional second urea sensor located downstream of the dialysate regeneration system.

In any embodiment, the sensors can comprise at least two of an ammonia sensor, an ammonium sensor, and a pH sensor located downstream of the urease and upstream of an ammonium and/or ammonia exchange material.

In any embodiment, the method can be performed by a dialysis system.

In any embodiment, the patient urea level at a beginning of the dialysis session can be estimated.

In any embodiment, the patient urea level at an end of the dialysis session can be estimated.

In any embodiment, the patient urea level at an arbitrary time during treatment can be estimated.

In any embodiment, the patient urea level can be estimated at a beginning of the dialysis session and at an end of the dialysis session.

In any embodiment, the method can comprise the step of estimating a urea reduction ratio and/or a dialysis adequacy based on calculations using the patient urea level at a beginning of a dialysis session and the patient urea level at the end of the dialysis session.

The features disclosed as being part of the second aspect of the invention can be in the second aspect of the invention, either alone or in combination.

BRIEF DESCRIPTION OF THE DRAWINGS

FIG. 1 is a flow chart of a method for estimating a patient urea level at any arbitrary time during treatment.
FIG. 2 is a non-limiting embodiment of a dialysis system.
FIG. 3 is a schematic of a system for estimating a patient urea level during treatment.
FIG. 4 is a schematic of a system for estimating a patient urea level during treatment with a single urea sensor.

DETAILED DESCRIPTION OF THE INVENTION

Unless defined otherwise, all technical and scientific terms used generally have the same meaning as commonly understood by one of ordinary skill in the art.

The articles "a" and "an" are used to refer to one or to over one (*i.e.*, to at least one) of the grammatical object of the article. For example, "an element" means one element or over one element.

A "ammonia sensor" can be any component capable of determining a concentration of ammonia within a fluid.

A "an ammonium and/or ammonia exchange material" can be any material capable of removing ammonium and/or ammonia from a solution. In certain embodiments, the ammonium and/or ammonia can be removed by exchanging the ammonia and/or ammonium with a different solute. Alternatively, the ammonium and/or ammonia exchange material can remove the ammonium and/or ammonia by any alternative means, and is not limiting to exchange with a different solute.

A "ammonium sensor" is any component capable of determining a concentration of ammonium ions within a fluid.

The term "amount of urea removed by the dialysate regeneration system" refers to a difference between an amount of urea entering a dialysate regeneration system and an amount of urea exiting the dialysate regeneration system.

The term "arbitrary time during treatment" can refer to any point in time during a dialysis session.

A "combined pH, ammonium, and/or ammonia sensor" is a single sensor capable of measuring any two or more of pH, ammonium concentration, and/or ammonia concentration.

The term "communication" refers to an electronic or wireless link between two components.

The term "comprising" includes, but is not limited to, whatever follows the word "comprising." Use of the term indicates the listed elements are required or mandatory but that other elements are optional and may be present.

The term "concentration" refers to an amount of a solute dissolved in a solvent.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The terms "determining" and "determine" can refer to ascertaining or identifying a particular state or desired state. As used in "determining significant parameters," the phrase refers to ascertaining or identifying any parameter. For example, a system or fluid, or any measured variable(s) or feature(s) of a system or a fluid can be determined by obtaining sensor data, retrieving data, performing a calculation, or by any other known method.

The term "dialysance" refers to a volume of blood cleared of a solute per unit of time.

The term "dialysate" can describe a fluid into or out of which solutes from a fluid to be dialyzed diffuse through a membrane. A dialysate typically can contain one or more electrolytes close to a physiological concentration of the electrolyte(s) found in blood.

The term "dialysate flow path" can refer to a fluid pathway or passageway that conveys a fluid, such as dialysate and is configured to form at least part of a fluid circuit for peritoneal dialysis, hemodialysis, hemofiltration, hemodiafiltration or ultrafiltration.

The term "dialysate regeneration system" refers to a set of components capable of removing solutes from a dialysate, allowing the dialysate to be reused.

The term "dialysis adequacy" is a measure of an amount of urea removed from a patient during treatment compared to a desired amount of urea to remove from the patient.

A "dialysis session" can be a period of time in which treatment of a patient by dialysis is ongoing.

The term "dialysis system" can refer to a set of components configured to carry out dialysis therapy of any type including peritoneal dialysis, hemodialysis, hemofiltration, hemodiafiltration, or ultrafiltration.

The term "dialyzer" can refer to a cartridge or container with two flow paths separated by semi-permeable membranes. One flow path is for blood and one flow path is for dialysate. The membranes can be in hollow fibers, flat sheets, or spiral wound or other conventional forms known to those of skill in the art. Membranes can be selected from any one or combination of materials: polysulfone, polyethersulfone, poly (methyl methacrylate), modified cellulose, or other materials known to those skilled in the art.

The term "downstream" can refer to a position of a first component in a flow path relative to a second component wherein fluid will pass by the second component prior to the first component during normal operation. The first component can be said to be "downstream" of the second component, while the second component is "upstream" of the first component.

The term "estimating" and "estimate" can refer to an approximation of a value for a particular parameter.

The term "flow rate" refers to the volume of fluid moving through a conduit or system per unit time.

The term "fluidly connectable," "fluidly connect," "for fluid connection," and the like, can refer to the ability of providing for the passage of fluid, gas, or a combination thereof, from one point to another point. The two points can be within or between any one or more of compartments, modules, systems, components, and rechargers, all of any type.

The connection can optionally be disconnected and then reconnected.

The term "generation rate" refers to the rate at which a substance is created from constituent parts within a body of a patient.

The term 'initiate a dialysis session" or "initiating a dialysis session" can refer to beginning a treatment of a patient by any type of dialysis.

A "lookup table" can be an electronic or non-electronic table correlating the effects of changing a particular variable or variables on an outcome.

The term "mass" refers to a measure of an amount of matter in a substance.

The term "mass transfer rate" is a measure of an amount of a substance that is moved in a given period of time.

A "mathematical model" is an algorithm or set of equations that provide a solution for at least one variable based on one or more input variables.

A "patient" or "subject" can be a member of any animal species, preferably a mammalian species, optionally a human. The subject can be an apparently healthy individual, an individual suffering from a disease, or an individual being treated for a disease.

The term "patient urea level" can refer to the amount of urea within the body of a patient. The urea level can refer to direct measurements of urea, or to measurement of patient blood urea nitrogen, which is a measure of nitrogen in the blood of a patient that comes from urea. The BUN measurement is given in units of mg/dl.

The term "pH sensor" refers to any component capable of measuring the hydrogen ion concentration in a fluid.

The term "processor" as used herein is a broad term and is to be given its ordinary and customary meaning to a person of ordinary skill in the art. The term refers without limitation to a computer system, state machine, processor, or the like designed to perform arithmetic or logic operations using logic circuitry that responds to and processes the basic instructions that drive a computer. The terms can include ROM ("read-only memory") and/or RAM ("random-access memory") associated therewith.

The term "production rate" refers to an amount of a substance created by chemical reactions in a given period of time.

The term "programmed," when used referring to a processor or computer, can refer to a series of instructions that cause a processor, software, hardware, or computer to perform certain steps.

The term "regenerated dialysate" refers to dialysate that has contacted blood across a dialyzer and has been treated to remove one or more solutes after contacting the blood.

"Solutions to" a formula refer to any values obtained using the formula, or derivatives of the formula. Derivatives of a formula can refer to any other formula that is obtained by algebraic or any other mathematical manipulation of the formula.

The terms "sorbent cartridge" and "sorbent container" can refer to a cartridge containing one or more sorbent materials for removing specific solutes from solution, such as urea. The term "sorbent cartridge" does not require the contents in the cartridge be sorbent based, and the contents of the sorbent cartridge can be any contents that can remove waste products from a dialysate. The sorbent cartridge may include any suitable amount of one or more sorbent materials. In certain instances, the term "sorbent cartridge" can refer to a cartridge which includes one or more sorbent materials in addition to one or more other materials capable of removing waste products from dialysate. "Sorbent cartridge" can include configurations where at least some materials in the cartridge do not act by mechanisms of adsorption or absorption.

The term "upstream" can refer to a position of a first component in a flow path relative to a second component wherein fluid will pass by the first component prior to the second component during normal operation. The first component can be said to be "upstream" of the second component, while the second component is "downstream" of the first component.

"Urea" is a compound with a chemical formula $(NH_3)_2CO$.

The term "urea reduction ratio" refers to the percentage of a patient's urea that is removed during treatment.

"Urease" is an enzyme that catalyzes the conversion of urea into carbon dioxide and ammonium ions.

A "urea sensor" is any component capable of determining a concentration of urea within a fluid.

*Pre-BUN Estimator*

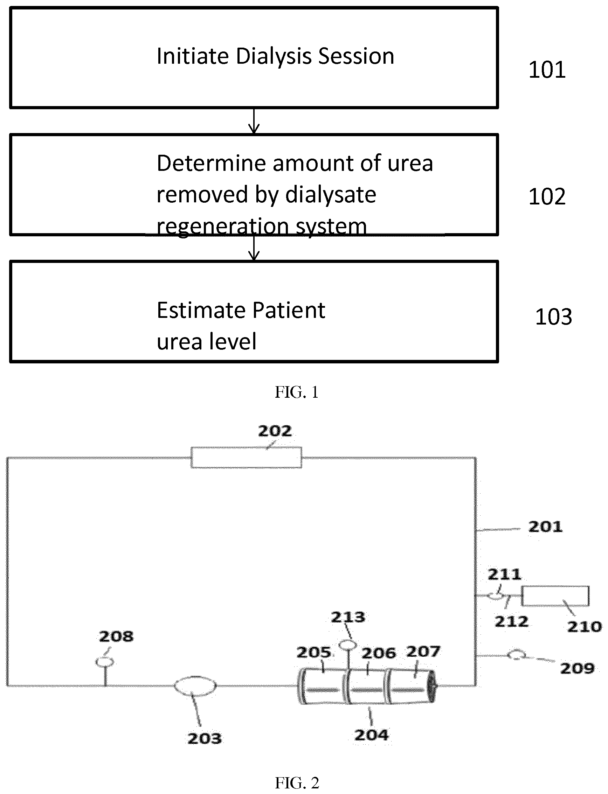

FIG. 1 is a flow chart showing the steps in estimating a patient urea level prior to a dialysis session. In step **101**, a dialysis session can be initiated. After initiating a dialysis session, an amount of urea removed by a dialysate regeneration system can be determined in step **102**. The dialysate regeneration system can include urease, which catalyzes the conversion of urea to ammonium ions and carbon dioxide. The relative concentrations of ammonium ions and ammonia in solution after conversion of urea by the urease varies with the pH of the dialysate. The ammonium ions can be exchanged for hydrogen or sodium ions with a ammonium and/or ammonia exchange material, such as zirconium phosphate prior to returning the dialysate to a dialyzer. The dialysate regeneration system can include one or more sorbent cartridges containing the urease, an ammonium and/or ammonia exchange material, and other sorbent materials to remove specific solutes from the dialysate. Alternatively, the dialysate regeneration system can include urease and an electrodialysis system that removes the generated ammonium ions from the dialysate.

Multiple methods of determining the amount of urea removed by the dialysate regeneration system can be used in step **102**. As a non-limiting example, the system can use one or more urea sensors in communication with a processor upstream and/or downstream of the dialysate regeneration system to determine the amount of urea removed from the dialysate. For example, first urea sensor can be located in any location in a dialysate flow path downstream of a dialyzer and upstream of the dialysate regeneration system. A second urea sensor can be located in any location in the dialysate flow path downstream of the urease and upstream of the dialyzer, including within the dialysate regeneration system. Alternatively, the amount of urea removed by the dialysate regeneration system can be determined by measuring any two of ammonia, ammonium ions, and pH of the dialysate at a location downstream of the urease and upstream of an ammonium and/or ammonia exchange material with sensors in communication with the processor. The total ammonia concentration is the sum of the concentrations of the ammonium ions and ammonia in the dialysate. Because the relative concentrations of ammonia and ammonium ions depends on the pH, the total ammonia can be determined by measuring any two of the pH, ammonia concentration, and ammonium ion concentration after conversion of urea and prior to removal of the ammonia and ammonium ions by the ammonium and/or ammonia exchange material. The amount of urea removed from the dialysate by the dialysate regeneration system will be one-half of the total ammonia produced.

In step **103**, a processor of the dialysis system can estimate the patient urea level based on the amount of urea removed by the sorbent regeneration system. The processor can be programmed to use mathematical models to estimate the patient urea level, or can use lookup tables. As described, the system can estimate the urea level of the patient at any arbitrary time before, during, and/or after treatment. As an example, the processor can be programmed to estimate the initial pre-treatment urea level of the patient, an ending post-treatment urea level of the patient, and/or a urea level of the patient at any time during treatment. Table 1 provides non-limiting uses of the patient urea level estimation, along with exemplary times during treatment at which the patient urea level can be estimated. One of skill in the art will understand that the urea level of the patient can be used by clinicians in additional ways not listed in Table 1.

Table 1

| Use | Time during treatment for estimation |
|---|---|
| Determination of dialysis adequacy | Beginning of treatment and end of treatment |
| Determination of likely dialysis adequacy | Beginning of treatment and any arbitrary time during treatment |

As illustrated in Table 1, the urea level of the patient can be estimated at the beginning of treatment and at the end of treatment to estimate a urea reduction ratio for the dialysis session. Based on the urea reduction ratio, the user can decide whether the dialysis session provided adequate treatment to the patient. If it has not, the current dialysis session can be extended, future dialysis sessions can be lengthened, and/or additional dialysis sessions can be scheduled. Similarly, the urea level of the patient can be estimated at the beginning of treatment as well as any arbitrary time during treatment to calculate an in-session urea reduction ratio. The in-session urea reduction ratio can indicate whether the current dialysis session is likely to provide adequate treatment. For example, the processor can be programmed to estimate the urea reduction ratio for the patient at any arbitrary time during treatment and compare the estimated urea reduction ratio to an expected urea reduction ratio at the same time during treatment. The algorithms described can provide an expected urea reduction ratio at any arbitrary time during treatment, and the expected urea reduction ratio at an arbitrary time can be compared to the estimated urea reduction ratio at the same time. If the expected urea reduction ratio is higher than the estimated urea reduction ratio, the dialysis session can be extended. If the expected urea reduction ratio is lower than the estimated urea reduction ratio, the dialysis session can be shortened. Further, the algorithms can be used to calculate a point in time during treatment when a specific urea reduction ratio will have been achieved to estimate a necessary dialysis session length for adequate treatment. For example, the algorithm can calculate the time at which the patient is expected to have a urea reduction ratio of 0.65 to estimate the proper dialysis session length.

FIG. 2 is a high level diagram of a dialysis system for estimating a patient urea level at any arbitrary time during treatment. Dialysate in a dialysate flow path **201** can contact blood in an extracorporeal flow path (not shown) across a semipermeable membrane in a dialyzer **202**. Solutes in the blood can cross the semipermeable membrane of the dialyzer **202** into the dialysate in the dialysate flow path **201**. Dialysate pump **203** provides a driving force for moving dialysate through the dialysate flow path **201**. Sorbent cartridge **204** can remove solutes from the dialysate in the dialysate flow path **201**, allowing the dialysate to be reused. As illustrated in FIG. 2, the sorbent cartridge **204** can include one or more sorbent modules containing sorbent materials, including a urease containing sorbent module **205**, an anion exchange resin, such as zirconium oxide, in a second sorbent module **206**, and an ammonium and/or ammonia exchange material, such as zirconium phosphate, in sorbent module **207**. One of skill in the art will understand that the sorbent materials can be contained in a single cartridge, or one or more separate sorbent cartridges or modules. The order of sorbent materials in sorbent cartridge **204** can also be modified, so long as a cation exchange resin is located downstream of the urease. As described, the system can use alternative dialysate regeneration systems to remove solutes from the dialysate in place of the sorbent cartridge **204**. Bicarbonate can be added to the dialysate flow path **201** from bicarbonate source **210** fluidly connected to dialysate flow path **201**. Bicarbonate pump **211** pumps a bicarbonate concentrate from the bicarbonate source **210** through conduit **212** and into the dialysate flow path **201** at a bicarbonate metering rate. Additional components not shown in FIG. 2 can also be present, including a degasser, a water source, a cation infusate source and any other components necessary for dialysis treatment.

Urea sensor **208** can measure the urea concentration in the dialysate upstream of sorbent cartridge **204**. Optionally, a second urea sensor **209** can be included in the dialysate flow path **201** to measure the urea concentration of the dialysate downstream of the sorbent cartridge **204**. The amount of urea removed by the sorbent cartridge **204** is the difference between the urea concentration upstream of the sorbent cartridge **204** and the urea concentration downstream of the sorbent cartridge **204**. Additional sensors can be included in the dialysate flow path **201** for improved accuracy. Alternatively, or additionally, the system can use a sensor **213** capable of measuring any two or more of pH, ammonia concentration, and ammonium ion concentration. Although shown as a single combined pH, ammonium, and/or ammonia sensor **213** in FIG. 2, one of skill in the art will understand that separate sensors can be used. The sensor **213** measuring pH, ammonia concentration, and/or ammonium ion concentration can be placed at any location between urease containing sorbent module **205** and zirconium phosphate containing sorbent module **207**. The sensor **213** can be placed inside of the sorbent cartridge **204**, or between sorbent modules or housings. By measuring at least two of pH, ammonia concentration, and ammonium ion concentration, the total ammonia concentration of the dialysate at the location of sensor **213** can be determined, which is equal to twice the concentration of urea converted by the sorbent cartridge **204**.

*Pre-BUN Estimation Algorithm*

An example mathematical description for arbitrary dissolved chemical species *"i"* in the patient by a dynamic mass balance is provided in Eq(1). The system can estimate the patient urea level using solutions to the formula provided in Eq(1). As described, the system can use solutions to formulas that are derivatives of the formula in Eq(1). For example, Eq's (15) and (16) can be derived from Eq(1) as described and solutions to Eq's (15) and (16) used in estimating the patient urea level.

$$\frac{dM_{P,i}}{dt} = G_{P,i} - (Ind)J_i(V_P, M_{P,i}, C_{Di,i}) + R_{P,i} \qquad \text{Eq(1)}$$

$M_{p,i}$ is the mass of the species *"i"* in the patient [mole], $G_{p,i}$ is the generation rate of the of the species *"i"* in the patient [mole/min], $J_i$ is the total mass transfer rate of species *"i"* from the patient into the dialysate [mole/min], $C_{Di,i}$ is the concentration of species *"i"* in the regenerated dialysate when the regenerated dialysate enters the dialyzer [M], and $R_{P,i}$ is the production rate of species *"i"* due to chemical reactions [mole/min], and *Ind* is a binary indicator variable for dialysis therapy with *Ind* = 0 if dialysis is not occurring, and *Ind* = 1 if dialysis is occurring.

In certain embodiments, the dialysis system uses urease to enzymatically convert an amount of urea to ammonium and carbonate in accordance with Eq(2).

$$x(Urea + 2H_2O) \xrightarrow{Urease} y(CO_3^{2-} + 2NH_4^+) + (x - y)(Urea + 2H_2O),\ y < x. \qquad \text{Eq(2)}$$

The amount of urea converted by the urease layer can be measured by urea sensors placed before and after the enzyme reactor. Alternatively, the ammonia and/or ammonium concentration immediately after the urease layer, with an associated pH, can be used as a marker for the change in urea concentration across the urease layer. Depending on which two of pH, ammonia, and ammonium concentration are used, the algorithm can calculate the amount of urea converted by the urease using Eq's (3)-(5) where $\Delta Ccol_{Urea}^{Sensor}$ is the amount of urea removed by the dialysate regeneration system and $K_{NH_3}$ is the equilibrium constant for the conversion of ammonia to ammonium ions.

The processor can receive the ammonia concentration, ammonium ion concentration, and/or pH, or alternatively the urea concentrations upstream, and optionally downstream, of the urease, and record the values along with the corresponding treatment time, *tCrit.* The treatment time can be any arbitrary time during a dialysis session, however, the earliest useful time for the algorithm may be when the ammonia or ammonium ion concentration leaving the urease layer is at a maximum shortly after treatment begins. The finite volume of the components in the dialysate flow path between the dialyzer and the urease layer V and the finite flow rate through the that volume Q imply a residence time T = V/Q. Assuming that treatment begins at t = 0, the earliest time that the products of the urease reaction will leave the urease layer and be measured by the sensor is 0 + T. The earliest time corresponds to an assumption that flow through the cartridge follows idealized plug-flow behavior (in this case, the ammonium sensor would see a step change from 0 to the maximum level expected during treatment). However, in practice, idealized plug-flow behavior is an approximation and the sensor measurement will gradually reach a maximum at time 0 + T + D, where D > 0 is a time related to how disperse the flow through the cartridge is. By measuring the ammonium or ammonia several times and monitoring for the maximum, which occurs near the beginning of treatment, one can reduce some of the error that may be caused in applying the extrapolations described in the algorithm.

$$\Delta Ccol_{Urea}^{Sensor} = \frac{1}{2}\left([NH_4^+] + [NH_3]\right) \qquad Eq(3)$$

$$\Delta Ccol_{Urea}^{Sensor} = \frac{1}{2}[NH_4^+]\left(1 + \frac{K_{NH_3}}{[H^+]}\right) \qquad Eq(4)$$

$$\Delta Ccol_{Urea}^{Sensor} = \frac{1}{2}[NH_3]\left(1 + \frac{[H^+]}{K_{NH_3}}\right) \qquad Eq(5)$$

By way of a mathematical model for enzyme kinetics, which can be a Michaelis-Menten model, and a minimization algorithm, the urease layer inlet $\left(Ccol_{Urea_{In}}^{Algo}\right)$ and outlet $\left(Ccol_{Urea_{Out}}^{Algo}\right)$ concentrations of urea can be calculated using the change in urea concentration across the urease layer by:

$$\min_{Ccol_{Urea_{In}}^{Algo}} \left|\Delta Ccol_{Urea}^{Algo} - \Delta Ccol_{Urea}^{Sensor}\right| \qquad Eq(6)$$

Eq(6) is used to minimize the absolute difference of changes in urea across the urease material in the sorbent regeneration system as measured by the described sensors and as calculated from the described algorithms by varying the guess of urea at the inlet of the urease layer in the algorithm. The minimization problem can be solved using a variety of minimization algorithms known in the art or by application of iterative methods. The change in urea as calculated from the described algorithm $\Delta Ccol_{Urea}^{Algo}$ is given by:

$$\Delta Ccol_{Urea}^{Algo} = Ccol_{Urea_{In}}^{Algo} - Ccol_{Urea_{Out}}^{Algo} \qquad Eq(7)$$

$Ccol_{Urea_{In}}^{Algo}$ and $Ccol_{Urea_{Out}}^{Algo}$ can be calculated using Eq's (8)-(10).

$$\frac{dC_{PFR}}{dV} = \left(\frac{1}{Q_{col}}\right)\left(\frac{d_{IU}NIU}{V_{PFR}}\right)\left(\frac{C_{PFR}}{K_m + C_{PFR}}\right), \; C(0) = Ccol_{Urea_{In}}^{Algo} \qquad Eq(8)$$

where:

$$\Delta Ccol_{Urea}^{Algo} = Ccol_{Urea_{In}}^{Algo} - Ccol_{Urea_{Out}}^{Algo} \qquad Eq(9)$$

and where

$$C_{PFR}(V_{PFR}) = Ccol_{Urea_{Out}}^{Algo} \qquad\qquad \text{Eq(10)}$$

The solution $C_{PFR}(V_{PFR})$ is insensitive to the value of $V_{PFR}$ as long as the value is held constant during the evaluation of Eq (9). The variable dIU is a chemical constant related to urease, and does not change (0.5e-6 mol/min/IU). This leaves variables Qcol (flow rate through the urease layer), NIU (amount of active urease in the layer), and Km (the

$$C(0) = Ccol_{Urea_{In}}^{Algo}$$

Michaelis-Menten constant of urease). The initial condition $C(0) = Ccol_{Urea_{In}}^{Algo}$ will also influence the amount of urea leaving the urease layer. As an example, for the case where Qcol = 500 mL/min, NIU = 30,000 IU, Km = 12 mM, and C(0) = 10 mM, we have $C_{PFR}$ = 1.65 mM, which is a reduction of 83.5% in urea concentration.

In the case of the beginning of dialysis treatment, the value of $Ccol_{Urea_{Out}}^{Algo}$ will be 0 until enough time has elapsed for urea to leave the urease layer. As such, a single urea sensor upstream of the dialysate regeneration system can be used to estimate the patient urea level without knowledge of the amount of urea removed by the dialysate regeneration system.

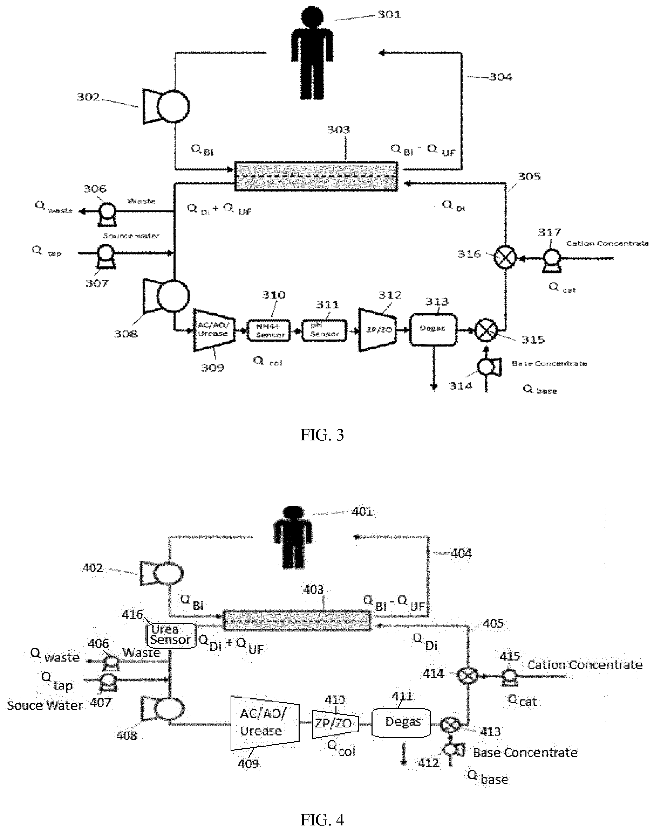

Once the urea concentrations entering and leaving the urease layer are known, mass balance equations can be evaluated using flow rates set on and/or reported by the device. FIG. 3 provides a schematic representation of the mass balance of fluid in the system. One of skill in the art will understand that the schematic of FIG. 3 is for illustrative purposes only. The dialysis system includes an extracorporeal flow path **304** fluidly connected to a dialyzer **303**. Blood from the patient **301** is pumped through the extracorporeal flow path **304** by blood pump **302**. Dialysate is pumped through dialysate flow path **305** by dialysate pump **308**. Waste or ultrafiltrate can be removed by waste pump **306** at a flow rate of Qwaste. Additional water can be added to the dialysate flow path **305** by water pump **307** at a flow rate of Qtap. The dialysate regeneration system can include a first module **309** containing urease, and optionally activated carbon and/or alumina oxide. The flow rate of fluid through the dialysate regeneration system is given as Qcol. An ammonium sensor **310** and a pH sensor **311** can be placed downstream of the first module **309**. Although FIG. 3 shows an ammonium sensor **310** and a pH sensor **311** positioned between the urease containing first module **309** and a zirconium phosphate containing sorbent module **312**, other sensors can be used including one or more urea sensors and/or an ammonia sensor. The sensors can be included in any order, and can include the pH sensor **311** upstream of the ammonium sensor **310** or an ammonia sensor. As described, sensors can be used to measure any two of pH, ammonia, and ammonium ions. Further, the order of the sensors can be modified from that shown in FIG. 3. One or more urea sensors (not shown) can also be used in place of, or in addition to, the pH, ammonia, and/or ammonium sensors. A degasser **313** can remove carbon dioxide formed from the breakdown of urea. Bicarbonate can be added from a bicarbonate source (not shown) by bicarbonate pump **314** at a bicarbonate addition rate of Qbase. A static mixer **315** can optionally be included to ensure complete mixing of the bicarbonate concentrate with the dialysate. Cation infusates, such as calcium, magnesium, and potassium can be added by cation concentrate pump **317** at a flow rate of Qcat. A static mixer **316** can optionally be included to ensure complete mixing of the cation concentrate with the dialysate. The flow rate of blood entering the dialyzer **303** in FIG. 3 is given as $Q_{Bi}$. The flow rate of blood exiting the dialyzer **303** will be $Q_{Bi}$ - $Q_{uf}$, where $Q_{uf}$ denotes the ultrafiltration rate. The flow rate of dialysate entering the dialyzer **303** is given as $Q_{Di}$. The flow rate of dialysate exiting the dialyzer **303** will be $Q_{Di}$ + $Q_{uf}$.

Using mass balance equations, the urea entering ($CDi_{Urea}$) and leaving ($CDo_{Urea}$) the dialyzer as well as in the patient at a critical time point $(CBi_{Urea}^{tCrit})$ can be calculated with Eq's (11)-(13). The urea entering and leaving the dialyzer can alternatively be measured directly with one or more urea sensors as opposed to using Eq's (11)-(12).

$$CDo_{Urea} = Ccol_{Urea_{In}}^{Algo} \left( \frac{Q_{Di} - Q_{base} - Q_{cat}}{Q_{Di} - Q_{base} - Q_{cat} - Q_{tap}} \right) \qquad\qquad \text{Eq(11)}$$

$$CDi_{Urea} = Ccol_{Urea_{Out}}^{Algo} \left( \frac{Q_{Di} - Q_{base} - Q_{cat}}{Q_{Di} - Q_{cat}} \right) \qquad\qquad \text{Eq(12)}$$

$$CBi_{Urea}^{tCrit} = \left( \frac{Q_{Di}}{D_{Urea}} \right) (CDo_{Urea} - CDi_{Urea}) + CDi_{Urea} \qquad\qquad \text{Eq(13)}$$

The algorithm uses the dialysance of urea $D_{Urea}$, which can be calculated using Eq(14). Alternatively, the dialysance of sodium can be used to approximate the clearance of urea. The clearance of sodium can be measured by the dialysis system using sodium pulses or by any other method known in the art.

$$D_{Urea} = (IVIC_{Urea}) \left[ Q_{Bi} \frac{e^{KoA\frac{(QDi-QBi)}{QDiQBi}} - 1}{e^{KoA\frac{(QDi-QBi)}{QDiQBi}} - \frac{QBi}{QDi}} \right] \qquad Eq(14)$$

In addition to calculating the urea level of the patient at any arbitrary time point, interpolation and/or extrapolation of sensor measurements and the calculations within, and/or predictive modeling can be used to estimate the urea level in the patient at other treatment times including, but not limited to, treatment start (t = 0) and treatment end (t = T) with Eq's (15)-(16).

$$CBi_{Urea}^0 = \frac{CBi_{Urea}^{tCrit}}{e^{-\frac{D_{Urea}tCrit}{V_{Urea}}}} \qquad Eq(15)$$

$$CBi_{Urea}^T = CBi_{Urea}^{tCrit} e^{-\frac{D_{Urea}T}{V_{Urea}}} \qquad Eq(16)$$

Eq's (14)-(15) assume a single-compartment model for urea in the patient without ultrafiltration to estimate patient urea, although one skilled in the art could apply more complex approaches such as a multicompartment patient model and/or equations accounting for ultrafiltration. Additionally, a model of the sorbent hemodialysis system with a connected patient undergoing hemodialysis treatment, such as one based on differential equations, could be used to account for cumulative and/or dynamic effects on the patient urea level at or up to an arbitrary time point, such as those due to a history of changing the blood flow rate during treatment.

If the amount of urease enzyme is sufficiently large in the sorbent system, Eq's 15 and 16 are a good approximation to estimate the urea concentration in the patient. In the event that the urease enzyme is small, a better estimate could be obtained by a mathematical model that implements the enzyme reactor differential equation provided in Eq (8) or various other alternatives in the art (such as those based on partial differential equations).

To avoid errors in estimation of patient urea caused by Eq's 15 and 16 for the recirculating dialysis modality due to enzyme reactor dependent effects, the change in urea across the reactor (urease layer) early in the treatment as the error between using Eq's 15 and 16 can be assessed and a more sophisticated model that combines the concepts of Eq 8 with Eq's 15 and 16 could accumulate as the treatment time passes. In practice, one could model the entire sorbent hemodialysis device with the patient connected to obtain a better estimate than Eq's 15 and 16.

Eq(17) is a derivation of the dialysance equation for an arbitrary species i. One of skill in the art will understand that either of the two equalities in Eq(17) can be used to calculate the dialysis and obtain Eq(13) for estimation of the patient urea level. In a preferred embodiment, the second equality in Eq(17) can be used, which eliminates the need to determine the blood outlet concentration.

$$D = \frac{Q_{Bi}(C_{Bi} - C_{Bo})}{C_{Bi} - C\_Di} = \frac{Q_{Di}(C_{Do} - C_{Di})}{C_{Bi} - C_{Di}} \qquad Eq(17)$$

FIG. 4 illustrates a system using a single urea sensor **416**. The dialysis system includes an extracorporeal flow path **404** fluidly connected to a dialyzer **403**. Blood from the patient **401** is pumped through the extracorporeal flow path **404** by blood pump **402**. Dialysate is pumped through dialysate flow path **405** by dialysate pump **408**. Waste or ultrafiltrate can be removed by waste pump **406** at a flow rate of Qwaste. Additional water can be added to the dialysate flow path **405** by water pump **407** at a flow rate of Qtap. The dialysate regeneration system can include a first module **409** containing urease, and optionally activated carbon and/or alumina oxide, and a second module **410** containing zirconium phosphate and zirconium oxide. The flow rate of fluid through the dialysate regeneration system is given as Qcol. A urea sensor **416** determines the urea concentration in the dialysate upstream of the dialysate regeneration system. A degasser **411** can remove carbon dioxide formed from the breakdown of urea. Bicarbonate can be added from a bicarbonate source (not shown) by bicarbonate pump **412** at a bicarbonate addition rate of Qbase. A static mixer **413** can optionally be included to ensure complete mixing of the bicarbonate concentrate with the dialysate. Cation infusates, such as calcium, magnesium, and potassium can be added by cation concentrate pump **415** at a flow rate of Qcat. A static mixer **414** can optionally be included to ensure complete mixing of the cation concentrate with the dialysate. The flow rate of blood entering the dialyzer **403** in FIG. 4 is given as $Q_{Bi}$. The flow rate of blood exiting the dialyzer **403** will be $Q_{Bi}$ - $Q_{uf}$, where $Q_{uf}$ denotes the ultrafiltration rate. The flow rate of dialysate entering the dialyzer **403** is given as $Q_{Di}$. The flow rate of dialysate exiting the dialyzer **403** will be $Q_{Di}$ + $Q_{uf}$. As described, at the beginning of treatment no urea will exit the dialysate regeneration system. As such, the value of $Ccol_{Urea_{Out}}^{Algo}$ will be 0 until enough time has elapsed for urea to leave the urease layer, allowing a single urea sensor **416** to be used in estimating the patient **401** urea level.

*Experiment 1*

The algorithm described herein was evaluated using the parameters listed in Table 2 for the case where ammonium and pH were measured post urease layer. Table 2 provides exemplary values for each of the parameters used, as well as the methods of obtaining the data for each parameter.

Table 2

| Parameter | Example Value | Description | Methods |
|---|---|---|---|
| QDi | 500 mL/min | Flow rate of dialysate at dialyzer inlet. | Known by device. |
| QBi | 300 mL/min | Flow rate of blood at dialyzer inlet. | Known by device. |
| Qbase | 5 mL/min | Flow rate of liquid bicarbonate infusate (0mL/min if dry powder is used). | Known by device. |
| Qcat | 2 mL/min | Flow rate of liquid cation infusate. | Known by device. |
| Qtap | 100mL/min | Flow rate of source water into dialysate loop volume. | Known by device. |
| KOA | 1645 mL/min | Dialyzer efficiency parameter derived from measured urea clearance. | Dialyzer property. |
| $d_{IU}$ | 0.5e-6 mol/min/IU | Conversion factor for urease IU to reaction rate. | Chemical constant. |
| NIU | 30,000 IU | Number of effective International Units of urease in single-use cartridge. | Sorbent cartridge parameter fixed at manufacturing. |
| Km | 12 mmol/L | Michaelis-Menten constant of urease enzyme. | Sorbent cartridge parameter fixed at manufacturing. |
| $V_{PFR}$ | 1.3 L | Volume of urease reactor. | Sorbent cartridge parameter fixed at manufacturing. |
| $IVIC_{Urea}$ | 0.85 | In vitro - in vivo correlation value for $D_{Urea}$. | Constant value can be assumed across patients. |
| $K_{NH3}$ | 10^(-9.25) mol/L | Acid dissociation constant for ammonium/ammonia. | Chemical constant. |
| $[NH_4^+]$ | 12 mmol/L | Ammonium concentration after single-use cartridge. | Measured by sensor. |
| $[NH_3]$ | 0.6748 mmol/L | Ammonia concentration after single-use cartridge. | Measured by sensor. |

(continued)

| Parameter | Example Value | Description | Methods |
|---|---|---|---|
| [H$^+$] | 1.0e-8 mmol/L | Hydrogen concentration after single-use cartridge. | Measured by sensor (pH). |
| tCrit | 15 min | Treatment time corresponding to sensor measurements. | Known by device. |
| V$_{Urea}$ | 40 L | Urea volume of distribution in the patient. | Can be measured by bioelectrical impedance, dose-response of heavy water (or other chemical markers), and/or calculated using anthropometric formulas such as the Watson equation. |

Using the values in Table 2, the described algorithm was run to estimate the patient pre-treatment urea level, as outlined in steps 1-8 below:

1. tCrit = 15 min, $[NH_4^+] = 0.012$ mol/L and [$H^+$] = 10$^{-8}$ mol/L.

2. 
$$\Delta Ccol_{Urea}^{Sensor} = \frac{1}{2}[NH_4^+]\left(1 + \frac{K_{NH_3}}{[H^+]}\right) = 0.0063 \text{ mol/L}.$$

3. 
$$\min_{Ccol_{Urea_{In}}^{Algo}} \left|\Delta Ccol_{Urea}^{Algo} - \Delta Ccol_{Urea}^{Sensor}\right| = 0.000037 \text{ mol/L}.$$

$$C_{PFR}(0) = Ccol_{Urea_{In}}^{Algo} = 0.0164773 \text{ mol/L} \quad \text{and} \quad C_{PFR}(V_{PFR}) = Ccol_{Urea_{Out}}^{Algo} = 0.0037655$$
mol/L.

4. 
$$CDo_{Urea} = Ccol_{Urea_{In}}^{Algo}\left(\frac{Q_{Di} - Q_{base} - Q_{cat}}{Q_{Di} - Q_{base} - Q_{cat} - Q_{tap}}\right) = 0.0207 \text{ mol/L}.$$

5. 
$$CDi_{Urea} = Ccol_{Urea_{Out}}^{Algo}\left(\frac{Q_{Di} - Q_{base} - Q_{cat}}{Q_{Di} - Q_{cat}}\right) = 0.0037 \text{ mol/L}.$$

6. 
$$D_{Urea} = (IVIC_{Urea})\left[Q_{Bi}\frac{e^{K0A\frac{(QDi-QBi)}{QDiQBi}} - 1}{e^{K0A\frac{(QDi-QBi)}{QDiQBi}} - \frac{QBi}{QDi}}\right] = 0.2428 \text{ L/min}.$$

7. 
$$CBi_{Urea}^{tCrit} = \left(\frac{QDi}{D_{Urea}}\right)(CDo_{Urea} - CDi_{Urea}) + CDi_{Urea} = 0.0386 \text{ mol/L}.$$

8. 
$$CBi_{Urea}^0 = \frac{CBi_{Urea}^{tCrit}}{e^{-\frac{D_{Urea}tCrit}{V_{Urea}}}} = 0.0423 \text{ mol/L}$$

Step 8 of the algorithm provides the estimated patient urea level at the beginning of treatment as 0.0423 mol/L. As described, the patient urea level can be calculated for any arbitrary time during treatment, including the beginning of treatment, the end of treatment, or any time point during treatment.

One skilled in the art will understand that various combinations and/or modifications and variations can be made in the described systems and methods depending upon the specific needs for operation. Moreover features illustrated or described as being part of an aspect of the invention may be used in the aspect of the invention, either alone or in combination.

The invention may be described by reference to the following numbered paragraphs:-

1. A dialysis system, comprising:

a dialysate flow path fluidly connectable to a dialyzer; the dialysate flow path having a dialysate regeneration system comprising urease;

either a urea sensor or at least two sensors selected from: ammonia sensors, ammonium sensors, and pH sensors; and
a processor; the processor in communication with the urea sensor, ammonia sensor, ammonium sensor, and/or pH sensor; the processor programmed to determine an amount of urea removed by the dialysate regeneration system;
the processor further programmed to estimate a patient urea level from either one or more lookup tables or a mathematical model based on the amount of urea removed by the dialysate regeneration system;
the mathematical model using solutions to a formula:

$$\frac{dM_{P,i}}{dt} = G_{P,i} - (Ind)J_i(V_P, M_{P,i}, C_{Di,i}) + R_{P,i}$$

, wherein $M_{p,i}$ is a mass of a species "i" in a patient, $G_{p,i}$ is a generation rate of the of the species "i" in the patient, $J_i$ is a total mass transfer rate of species "i" from the patient into a dialysate, $C_{Di,i}$ is a concentration of species "i" in a regenerated dialysate when the regenerated dialysate enters the dialyzer, $R_{P,i}$ is a production rate of species "i" due to chemical reactions, and $Ind$ is a binary indicator variable for dialysis therapy with $Ind = 0$ if dialysis is not occurring, and $Ind = 1$ if dialysis is occurring.

2. The dialysis system of paragraph 1, wherein the mathematical model uses a formula:

$$CBi_{Urea}^{tCrit} = \left(\frac{QDi}{D_{Urea}}\right)(CDo_{Urea} - CDi_{Urea}) + CDi_{Urea};$$ wherein $CBi_{Urea}^{tCrit}$ is the patient urea level at time t; $QDi$ is a dialysate flow rate exiting the dialyzer; $D_{Urea}$ is a dialysance of urea; $CDo_{Urea}$ is a urea concentration in a dialysate exiting the dialyzer; and $CDi_{Urea}$ is a urea concentration in the dialysate entering the dialyzer.

3. The dialysis system of paragraph 1, wherein the dialysate regeneration system comprises one or more sorbent cartridges.

4. The dialysis system of paragraph 1, wherein the sensors comprise a first urea sensor located upstream of the dialysate regeneration system and an optional second urea sensor located downstream of the dialysate regeneration system.

5. The dialysis system of paragraph 1, wherein the sensors comprise at least two of an ammonia sensor, an ammonium sensor, and a pH sensor located downstream of the urease and upstream of an ammonium and/or ammonia exchange material.

6. The dialysis system of paragraph 5, wherein the sensors are part of a combined pH, ammonium, and/or ammonia sensor.

7. The dialysis system of paragraph 1, the processor further programmed to estimate a urea reduction ratio based on a urea level of the patient at a beginning of a dialysis session and a urea level of a patient at an end of the dialysis session.

8. The dialysis system of paragraph 1, the processor further programmed to estimate a urea reduction ratio at an arbitrary time during treatment.

9. The dialysis system of paragraph 1, wherein the dialysis system has a single urea sensor upstream of the dialysate regeneration system.

10. The dialysis system of paragraph 9, the processor programmed to estimate the patient urea level at a beginning of a dialysis session.

11. A method, comprising:

a) initiating a dialysis session for a patient using a dialysis system having a dialysate regeneration system comprising urease in a dialysate flow path;
b) determining an amount of urea removed by the dialysate regeneration system based on data received from either one or more urea sensors or at least two sensors selected from: ammonia sensors, ammonium sensors, and pH sensors;
c) estimating a patient urea level from a mathematical model or a lookup table based on the amount of urea removed by the dialysate regeneration system; the mathematical model using solutions to a formula:

$$\frac{dM_{P,i}}{dt} = G_{P,i} - (Ind)J_i(V_P, M_{P,i}, C_{Di,i}) + R_{P,i}$$

, wherein $M_{p,i}$ is a mass of a species "i" in the patient, $G_{p,i}$ is a generation rate of the of the species "i" in the patient, $J_i$ is a total mass transfer rate of species "i" from the patient into a dialysate, $C_{Di,i}$ is a concentration of species "i" in a regenerated dialysate when the regenerated dialysate enters a dialyzer, $R_{P,i}$ is a production rate of species *"i"* due to chemical reactions, and *Ind* is a binary indicator variable for dialysis therapy with *Ind* = 0 if dialysis is not occurring, and *Ind* = 1 if dialysis is occurring.

12. The method of paragraph 11, wherein the mathematical model uses a formula:

$$CBi_{Urea}^{tCrit} = \left(\frac{QDi}{D_{Urea}}\right)(CDo_{Urea} - CDi_{Urea}) + CDi_{Urea};$$ wherein $CBi_{Urea}^{tCrit}$ is the patient urea level at time t; *QDi* is a dialysate flow rate exiting the dialyzer; $D_{Urea}$ is a dialysance of urea; $CDo_{Urea}$ is a urea concentration in a dialysate exiting the dialyzer; and $CDi_{Urea}$ is a urea concentration in the dialysate entering the dialyzer.

13. The method of paragraph 11, wherein the sensors comprise a first urea sensor located upstream of the dialysate regeneration system and a second urea sensor located downstream of the dialysate regeneration system.

14. The method of paragraph 11, wherein the sensors comprise at least two of an ammonia sensor, an ammonium sensor, and a pH sensor located downstream of the urease and upstream of an ammonium and/or ammonia exchange material.

15. The method of paragraph 11, wherein the method is performed by a dialysis system.

16. The method of paragraph 11, wherein the patient urea level is estimated at a beginning of the dialysis session.

17. The method of paragraph 11, wherein the patient urea level is estimated at an end of the dialysis session.

18. The method of paragraph 11, wherein the patient urea level is estimated at an arbitrary time during treatment.

19. The method of paragraph 11, wherein the patient urea level is estimated at a beginning of the dialysis session and at an end of the dialysis session.

20. The method of paragraph 19, further comprising the step of estimating a urea reduction ratio and/or a dialysis adequacy based on calculations using the patient urea level at a beginning of a dialysis session and the patient urea level at the end of the dialysis session.

**Claims**

1. A dialysis system, comprising:

   a dialysate flow path fluidly connectable to a dialyzer; the dialysate flow path having a dialysate regeneration system comprising urease;
   either a urea sensor or at least two sensors selected from: ammonia sensors, ammonium sensors, and pH sensors; and
   a processor; the processor in communication with the urea sensor, ammonia sensor, ammonium sensor, and/or pH sensor; the processor programmed to determine an amount of urea removed by the dialysate regeneration system;
   the processor further programmed to estimate a patient urea level from either one or more lookup tables or a mathematical model based on the amount of urea removed by the dialysate regeneration system;

   the mathematical model using solutions to a formula: $$\frac{dM_{P,i}}{dt} = G_{P,i} - (Ind)J_i(V_P, M_{P,i}, C_{Di,i}) + R_{P,i}$$ , wherein $M_{p,i}$ is a mass of a species *"i"* in a patient, $G_{p,i}$ is a generation rate of the of the species *"i"* in the patient, $J_i$ is a total mass transfer rate of species *"i"* from the patient into a dialysate, $C_{Di,i}$ is a concentration of species *"i"* in a regenerated dialysate when the regenerated dialysate enters the dialyzer, $R_{P,i}$ is a production rate of species *"i"* due to chemical reactions, and *Ind* is a binary indicator variable for dialysis therapy with *Ind* = 0 if dialysis is not occurring, and *Ind* = 1 if dialysis is occurring.

2. The dialysis system of claim 1, wherein the mathematical model uses a formula:

$$CBi_{Urea}^{tCrit} = \left(\frac{QDi}{D_{Urea}}\right)(CDo_{Urea} - CDi_{Urea}) + CDi_{Urea};$$ wherein $CBi_{Urea}^{tCrit}$ is the patient urea level at time t; *QDi* is a dialysate flow rate exiting the dialyzer; $D_{Urea}$ is a dialysance of urea; $CDo_{Urea}$ is a urea concentration in a dialysate exiting the dialyzer; and $CDi_{Urea}$ is a urea concentration in the dialysate entering the dialyzer.

3. The dialysis system of claim 1 or claim 2, wherein the dialysate regeneration system comprises one or more sorbent cartridges.

4. The dialysis system of any preceding claim, wherein the sensors comprise a first urea sensor located upstream of the dialysate regeneration system and an optional second urea sensor located downstream of the dialysate regeneration system.

5. The dialysis system of any preceding claim, wherein the sensors comprise at least two of an ammonia sensor, an ammonium sensor, and a pH sensor located downstream of the urease and upstream of an ammonium and/or ammonia exchange material.

6. The dialysis system of claim 5, wherein the sensors are part of a combined pH, ammonium, and/or ammonia sensor.

7. The dialysis system of any preceding claim, the processor further programmed to estimate a urea reduction ratio based on a urea level of the patient at a beginning of a dialysis session and a urea level of a patient at an end of the dialysis session.

8. The dialysis system of any preceding claim, the processor further programmed to estimate a urea reduction ratio at an arbitrary time during treatment.

9. The dialysis system of any preceding claim , wherein the dialysis system has a single urea sensor upstream of the dialysate regeneration system.

10. The dialysis system of claim 9, the processor programmed to estimate the patient urea level at a beginning of a dialysis session.

Initiate Dialysis Session

101

Determine amount of urea removed by dialysate regeneration system

102

Estimate Patient urea level

103

FIG. 1

**202**

**201**

**211**

**212** **210**

**213**

**208**

**205** **206 207**

**209**

**203**

**204**

FIG. 2

FIG. 3

FIG. 4

Europäisches
Patentamt

European
Patent Office

Office européen
des brevets

# EUROPEAN SEARCH REPORT

Application Number

EP 18 20 4839

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 95/03839 A1 (KHURI RAJA N [US]) 9 February 1995 (1995-02-09) * the whole document * * page 3 - page 4 * ----- | 1-10 | INV. A61M1/16 |
| Y | US 2014/158588 A1 (PUDIL BRYANT J [US] ET AL) 12 June 2014 (2014-06-12) * the whole document * * paragraphs [0004], [0281], [0283], [0289], [0310]; figure 1 * ----- | 1-4,7-10 | |
| Y | US 2015/367057 A1 (GERBER MARTIN T [US] ET AL) 24 December 2015 (2015-12-24) * the whole document * * paragraphs [0010], [0107]; figure 3 * ----- | 1-3,5-8, 10 | |
| A | EP 3 031 482 A1 (MEDTRONIC INC [US]) 15 June 2016 (2016-06-15) * the whole document * * paragraph [0089]; figure 2 * ----- | 1-10 | |
| A | EP 2 694 127 A2 (FRESENIUS MED CARE HLDG INC [US]) 12 February 2014 (2014-02-12) * the whole document * * paragraphs [0097], [0098], [0100]; figures 1, 3 * ----- | 1-10 | TECHNICAL FIELDS SEARCHED (IPC) A61M |

The present search report has been drawn up for all claims

| Place of search | Date of completion of the search | Examiner |
|---|---|---|
| The Hague | 11 March 2019 | Pinta, Violaine |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**

EP 18 20 4839

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2019

| Patent document cited in search report | | | Publication date | Patent family member(s) | | | Publication date |
|---|---|---|---|---|---|---|---|
| WO 9503839 | A1 | | 09-02-1995 | US | 5308315 | A | 03-05-1994 |
| | | | | US | 5405315 | A | 11-04-1995 |
| | | | | WO | 9503839 | A1 | 09-02-1995 |
| US 2014158588 | A1 | | 12-06-2014 | EP | 2950844 | A1 | 09-12-2015 |
| | | | | EP | 3219341 | A1 | 20-09-2017 |
| | | | | US | 2014158588 | A1 | 12-06-2014 |
| | | | | WO | 2014121167 | A1 | 07-08-2014 |
| US 2015367057 | A1 | | 24-12-2015 | EP | 3160536 | A1 | 03-05-2017 |
| | | | | US | 2015367057 | A1 | 24-12-2015 |
| | | | | WO | 2015199767 | A1 | 30-12-2015 |
| EP 3031482 | A1 | | 15-06-2016 | CN | 105688300 | A | 22-06-2016 |
| | | | | EP | 3031482 | A1 | 15-06-2016 |
| | | | | US | 2016166753 | A1 | 16-06-2016 |
| EP 2694127 | A2 | | 12-02-2014 | CA | 2832127 | A1 | 11-10-2012 |
| | | | | EP | 2694127 | A2 | 12-02-2014 |
| | | | | MX | 354890 | B | 22-03-2018 |
| | | | | US | 2012258545 | A1 | 11-10-2012 |
| | | | | US | 2015204831 | A1 | 23-07-2015 |
| | | | | WO | 2012138604 | A2 | 11-10-2012 |

EPO FORM P0459

For more details about this annex : see Official Journal of the European Patent Office, No. 12/82

**EP 3 482 784 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62583356 A